# EUROPEAN PATENT SPECIFICATION

(11) **EP 2 240 160 B1**
(45) Date of publication and mention of the grant of the patent: **18.12.2013**
(21) Application number: 09700172.1
(22) Date of filing: 07.01.2009
(51) Int. Cl.: A61K 9/12

(54) **LUBRICANT MOUSSE**
SCHMIERMITTELSCHAUM
MOUSSE LUBRIFIANTE

(30) Priority: 09.01.2008 GB 0800342
(43) Date of publication of application: 20.10.2010
(73) Proprietor: LRC Products Limited, Slough, Berkshire SL1 3UH (GB)
(72) Inventor: BRODIN, Christophe, Manchester M41 7HA (GB); JENKINSON, Andrew, London EC4V 6BW (GB)
(74) Representative: Carlin, Robert George
(86) International application number: PCT/GB2009/000024
(87) International publication number: WO 2009/087370

(56) References cited:
- WO-A-96/17595
- WO-A-2006/050951
- WO-A-2007/054818
- US-A- 4 310 510
- US-A- 4 913 893
- US-A1- 2005 095 272
- US-A1- 2007 224 149
- US-B1- 6 509 311

## Description

The present invention relates generally to mousses, particularly but not exclusively to massage and personal lubricant mousses, and to a method of making them.

Personal lubricants are well known. Personal lubricants are specialised lubricants which serve to reduce friction with body tissues. In particular, personal lubricants may be used to provide lubrication, or slip, during sexual activity. For example, personal lubricants can be used to increase pleasure or reduce pain during sexual intercourse, and can aid in reducing vaginal dryness.

A wide variety of personal lubricants is currently available. These lubricants generally function by supplying water to a body surface in a gelled or viscous form, by including a water-soluble polymer such as a water-soluble cellulose derivative or other water-soluble polymers, such as polyvinyl pyrrolidone, polyvinyl alcohol and the like. In use on a body surface, these systems retain a supply of water on the body surface, and water provides lubrication or slip. One or more humectants may be added to aid water retention, and thereby increase the lubrication provided by the lubricant and/or increase the longevity of the lubrication.

In addition to the known water-based lubricants, oil-based lubricants are known. In oil-based lubricants, the oily component itself provides lubrication (rather than functioning to retain water). However, a significant disadvantage of oil-based lubricants is that they are not compatible with all condom types. In particular, oil-based lubricants are not compatible with natural rubber latex condoms because oils degrade natural rubber latex. Accordingly, oil-based lubricants may cause defects in condoms, which is likely to reduce the effectiveness of latex condoms as a contraceptive and as protection against sexually transmitted diseases. This is a significant disadvantage of oil-based lubricants, as latex condoms are the most commonly used type of condom.

In addition to personal lubricants, massage lubricants are known. Massage lubricants include massage oils and lotions. However, these products are generally not intended for use as personal lubricants. Indeed, many of these products are not suitable for use as personal lubricants, because, for example, they tend to be oil based and therefore are not compatible with condoms.

In addition to the massage lubricants and personal lubricants discussed above, products that function both as a massage lubricant and as a personal lubricant (that is, 2-in-1 massage and personal lubricant formulations) are also known. The dual function formulation currently available include Durex Play® Massage Gel and Personal Lubricant (SSL International plc); Astroglide® 2-in-1 Warming Body Massage and Personal Lubricant (BioFilm, Inc); Eros Pjur® Essentials Original Personal Lubricant and Body Massage (Pjur Group Luxembourg SA) and K-Y Touch® Massage 2-in-1 Warming Body Massage Plus Personal Lubricant (Johnson and Johnson). The known massage and personal lubricants (or 2-in-1 lubricants) are either silicone based (Eros-Pjur) or water-based. They are all gel or lotion formulations.

Although 2-in-1 massage and personal lubricants are known, 2-in-1 massage and personal lubricants in the form of a mousse are not well known.

Mousse type products are known generally in the field of cosmetics and toiletries. Products in the form of a mousse are common in the field of hair care and cosmetic products. However, these products are not intended for use as personal lubricants, nor are they suitable for use as personal lubricants.

Massage products formulated as mousses (massage mousses) are known. However, these are intended for use as massage lubricants only, so have very different properties from 2-in-1 massage and personal lubricants. For example, the majority of massage mousses are primarily intended as skin moisturisers, so, in contrast to personal lubricants, they are designed to be absorbed by the skin. Therefore, any lubricity provided by these massage mousse products is short-lived and insufficient for use as a personal lubricant (personal lubricants suitably provide relatively high levels of lubricity, and suitably the lubricity is long lasting). In addition, massage mousses are not intended for use with condoms: they are, therefore, usually not compatible with condoms.

At present, there is one massage and personal lubricant mousse product on the market: Replens® Intimate Options Massage and Personal Lubricant Mousse, also marketed as Replens® Intimate Options Personal Lubricant Mousse (Lil' drug store Products, Inc). The listed ingredients include water, glycerol, isobutene, polyquaternium 7, PEG 8 stearate, propane, sodium stearate, polyquaternium 15, sucralose, flavour, methylparaben, propylparaben and potassium sorbate. However, this product suffers from several disadvantages: we have found that some of its properties are unsatisfactory. For example, the mousse is very stable. As a result, the mousse is not readily converted into the base lubricant in use, because the mousse is too stable. However, once the mousse does collapse, the resulting lubricant is of low viscosity, so it flows off the skin and/or away from the area to which it has been applied. The resulting lubricant is therefore unsatisfactory.

Accordingly, we have recognised a need for a massage and personal lubricant mousse which has satisfactory properties as both a personal lubricant and a massage lubricant, preferably a 2-in-1 lubricant mousse which has properties which are comparable to or better than 2-in-1 (non-mousse) products.

There are numerous difficulties inherent in formulating a 2-in-1 lubricant mousse. Formulating a mousse which has acceptable lubricant properties is problematic, because formulating a lubricant composition as a mousse increases the surface area of the composition, thereby increasing the rate at which the composition dries. As outlined above, lubricants generally function by retaining water on a body surface, which provides lubrication, or slip, at the surface. Therefore, increasing the surface area of a lubricant composition by formulating it as a mousse would be expected to be deleterious in terms of the lubrication provided by the lubricant, as the increased surface area results in a more rapid loss of water (and therefore a loss of lubricity) from the composition. It is, therefore, difficult to obtain a mousse formulation which is also lubricious.

Conversely, formulating a lubricant which is capable of forming an acceptable mousse is also problematic, because traditional mousse formulations and lubricants are typically of very different viscosities.

In contrast to the known massage mousses, a 2-in-i massage and personal lubricant mousse requires very particular properties: it must be both a good massage lubricant and a good personal lubricant. In particular, a balance of viscosity and mousse stability is required.

We have recognised that it would be advantageous to formulate a massage and personal lubricant (a 2-in-1 lubricant) in the form of a mousse, which has satisfactory properties in use. We have overcome the difficulties in formulating a massage and personal lubricant mousse with acceptable properties.

In its broadest aspect, the present invention provides a mousse comprising a propellant and a lubricant composition capable of being formed into a mousse, which composition is suitable for use as both a massage lubricant and a personal lubricant, wherein the lubricant composition comprises a lubricant base which is a gel, from 0.5% w/w to 5% w/w thickener comprising propylene glycol alginate, from 10% w/w to 30% w/w of one or more humectants / slip agents, and a carrier.

There is a massage and personal lubricant composition capable of being formed into a mousse, which composition comprises a lubricant base which is a gel. A "massage and personal" lubricant composition as used herein means a composition which is capable of performing satisfactorily as both a personal lubricant and a massage lubricant.

There is a massage and personal lubricant composition capable of being formed into a mousse, which composition comprises a lubricant base having a degassed viscosity of from 50 cP to 2000 cP. In a preferred aspect, the composition comprises a lubricant base having a degassed viscosity of from 500 cP to 2000 cP. In a preferred embodiment, the lubricant base has a degassed viscosity of from 750 cP to 2000 cP, more preferably from 1000 cP to 2000 cP. Preferably, the viscosity is from about 500 oP to about 1500 oP.

There is a massage and personal lubricant composition capable of being formed into a mousse, which composition comprises one or more humectants and slip agents; a thickener; and a carrier. Preferably, the carrier comprises an aqueous carrier such as water. Further components, for example one or more preservatives, may optionally be included.

There is also provided a container comprising a propellant and a lubricant composition capable of being formed into a mousse, which composition is suitable for use as both a massage lubricant and a personal lubricant, wherein the lubricant composition comprises a lubricant base which is a gel, from 0.5% w/w to 5% w/w thickener comprising propylene glycol alginate, form 10% w/w to 30% w/w of one or more humectants/slip agents, and a carrier.

The invention also provides a method of making a massage ane personal lubricant composition according to the invention, which method comprises providing a lubricant composition according to the invention; filling the composition into a container; and pressurising the container with propellant. Suitably, the lubricant composition is a gel. The container may, for example, be an aerosol-type can.

In a preferred aspect, there is provided a method of making a massage and personal lubricant composition, which method comprises providing a lubricant base formulation having a viscosity of from 50 cP to 2000 cP; filling the lubricant base formulation into a container; and pressurising with propellant.

The advantages of a 2-in-1 lubricant in the form of a mousse include ease of application, including less messy application; increased accuracy of application; reduced likelihood of spillage; and improved sensation on application and in use.

The compositions that form the mousse of the invention have several advantages, and are capable of forming a superior 2-in-1 massage and personal lubricant mousse. They form mousses of sufficient stability to be applied and for the massaging-in to be initiated before the mousse breaks down. However, thereafter the mousse breaks down fairly rapidly, so as to form time lubricant (i.e the mousse is stable for only a short period), In addition, once the mousse has collapsed, or been de-aerated, the composition forms a personal lubricant gel with highly satisfactory properties: it has extended longevity as a lubricant which does not dry out quickly, and exhibits minimal residue and tack in use.

As used herein, the term mousse includes dispersions of a gas, such as propellant or air, in a liquid. Dispersions of air in a liquid include colloids. Mousse products are typically dispensed from pressurised aerosol containers.

The terms "2-in-1 lubricant mousse" and "massage and personal lubricant mousse" are used interchangeably. As used herein, these terms mean a lubricant composition which is suitable for use both as a massage lubricant and a personal lubricant and which can be formulated as a mousse.

As used herein, unless otherwise indicated, the term viscosity generally refers to the initial lubricant viscosity of the formulation (that is, the viscosity of the lubricant base formulation, prior to forming it into a mousse).

Generally, the method of manufacturing a mousse formulation involves providing a base formulation; filling the base formulation into a container, such as a can; and pressurising with propellant. Therefore, in order to make a lubricant mousse, such as a massage and personal lubricant mousse, it is necessary to provide a lubricant base formulation; fill the lubricant base formulation into a container; and pressurise the container with propellant. The 2-in-1 lubricant mousse must therefore comprise a base formulation which is capable of forming an acceptable mousse. In contrast to standard personal lubricant formulations, this requirement places significant constraints on the base formulation, as not all base formulations are capable of forming mousses, and we have found that still fewer form acceptable mousses. For example, as the viscosity of the lubricant base increases, we have found that it becomes increasingly difficult to form a mousse. Typically, massage lubricants have viscosities of around 4,000 to 6,000 centipoise (cP) and therefore cannot be formed into mousses. Lower viscosities are required. However, if the viscosity of the lubricant base is too low, we have found that any mousse formed tends to be unstable and therefore unsatisfactory, unless high levels of surfactants, or foam stablilisers, are used. However, high levels of surfactants are undesirable: they can have detrimental effects on product safety; leave a residue on the skin as the lubricant dries; and can cause the lubricant to become tacky as it dries. Many users report dissatisfaction with residues and tackiness. In addition, when the viscosity of the lubricant base formulation is too low, it will not function satisfactorily as a personal lubricant, because it may run off the skin too readily, and may be absorbed into the skin too rapidly, such that the amount of lubricant needs to be topped up, or replenished, frequently during use. Users report that a need to repeatedly replenish a lubricant composition in use is inconvenient and disruptive.

In summary, therefore, a useable formulation must have a low enough viscosity to form a mousse, and the viscosity must be both sufficiently high that the formulation stays on the skin once the mousse has collapsed (i.e. converted into the lubricant base, or degassed) yet sufficiently low enough that, in use, the formulation provides the requisite lubricity.

We have found that the viscosity of the lubricant base formulation is an important feature: only a certain range of viscosities are suitable. Suitably, the lubricant formulation has a viscosity which is suitable for use as a personal lubricant and suitable for forming a mousse. Preferably, the viscosity of the formulation is from 50 cP to 2000 cP. More preferably, the viscosity is from 500 cP to 1500 cP. It is preferred that the lubricant formulation comprises a gel formulation, rather than, for example, a lotion or solution.

The 2-in-1 lubricant mousse is required to be sufficiently lubricious and long-lasting to act as a personal lubricant. Therefore, the formulation is preferably optimised such that it retains water during the mousse phase, and also such that it does not lose lubricity or become tacky as it starts to dry out.

The lubricant base formulation is preferably not thixotropic. That is, the formulation should exhibit only minimal shear thinning during application, because excessive shear thinning could result in the lubricant running off the skin.

The mousse is preferably sufficiently stable to allow application to the skin, yet sufficiently unstable to allow the mousse to collapse quickly to form the base lubricant, once the user starts to massage the mousse into the skin.

In addition, a 2-in-1 lubricant mousse is preferably condom-compatible. Positive aesthetics, such as a pleasant smell and appearance, are also highly preferred. Suitably, the final mousse composition (i.e. after the base lubricant composition has been formed into a mousse) comprises a slip agent and humectant; a thickener; a carrier phase; and a propellant.

The slip agent and humectant provide slip properties, or lubricity, and reduce the rate of water loss from the composition in use - for example, they reduce the rate of water loss from the mousse and the lubricant as it is massaged in to the skin. Any suitable slip agent and humectant, or mixtures thereof, can be used. Suitably the slip agent/humectant functions both as a slip agent and as a humectant. Preferred slip agents and humectants include, but are not limited to, polyhydric alcohols such as glycols, preferably low molecular weight glycols; polyethylene glycols, preferably high molecular weight polyethylene glycols; and high molecular weight polypropylene glycols. By high molecular weight we mean to include, for example, molecular weights of about 20kDa and above.

The slip agent and humectant can be included in any suitable amount. Suitable amounts of slip agent/humectant include amounts from about 10% w/w to about 30 % w/w, preferably from about 15% w/w to about 25% w/w, more preferably about 20% w/w.

The thickener comprises propylene glycol alginate.

The amount of thickener is suitably selected to optimise the viscosity of the composition. For example, the thickener may be present in an amount to give a viscosity (of the degassed lubricant base) of from about 50 cP to about 2000 cP. Preferably, the thickener is present in an amount to give the degassed lubricant base a viscosity of from about 500 cP to about 1500 cP. Suitable amounts include from about 0.5% w/w to about 5% w/w, preferably from about 0.5% w/w to about 2.5 % w/w, more preferably from about 1% w/w to about 2% w/w.

Any suitable carrier phase can be employed. However, it is highly preferred that the carrier phase is aqueous. It preferably comprises water. Water-based formulations are particularly preferred because they are compatible with condoms, particularly natural rubber latex condoms.

The propellant enables the lubricant base formulation to form a mousse. Preferably, the lubricant base formulation comprises a lubricant gel. Accordingly, the propellant preferably enables a lubricant gel base formulation to form a mousse. Any suitable propellant can be used. Preferred propellants include butane, isobutene, propane, compressed air, nitrogen and dimethylether. Butane is particularly preferred.

The propellant is suitably used in an amount to form a satisfactory mousse. Any suitable amount of propellant may be used. For example, amounts of propellant of about 10% w/w and below may be used. A preferred amount of propellant is from about 1% w/w to about 9% w/w, more preferably from about 2.5% w/w to about 7.5% w/w. A particularly preferred amount of propellant is about 5% w/w.

In addition to a slip agent and humectant; a thickener; a carrier phase; and (in the form of a mousse) a propellant, the composition optionally further comprises one or more additional components, for example to impart desirable properties to the composition. Optionally, the composition further comprises one or more of a moisturiser; an anti-tack agent; a foam booster and/or stabiliser; pH adjuster; flavourings and preservatives. Preferably, the composition comprises a moisturiser; an anti-tack agent; a foam booster and/or stabiliser; one or more flavourings and one or more preservatives.

The moisturiser, or emollient, where present, improves skin feel and skin benefit. For example, the moisturiser may give improved skin elasticity after use of the composition. Aloe vera is a preferred moisturiser. However, any suitable moisturiser may be used. The moisturiser may be used in any suitable amount. Amounts of up to about 5% w/w are preferred.

The composition optionally further comprises one or more anti-tack agents. An anti-tack agent counters any tackiness that may develop as the composition dries, for example as the composition dries on the surface of the user's skin. Any suitable anti-tack agent may be used. Preferred anti-tack agents include, but are not limited to, polyethyleneglycol (PEG) dimethicones such as, for example, PEG-12 dimethicone; and polytetrafluoroethylene (PTFE). Any suitable amount of anti-tack agent may be included. A suitable amount of anti-tack agent is, for example, about 1% w/w or below. Amounts of about 1% w/w or about 0.5 % w/w are preferred.

Optionally, the composition further comprises a foam stabiliser and/or foam booster. A foam booster/stabiliser preferably assists in the formation, stability and longevity of the mousse. Any suitable foam booster/stabiliser may be used. Preferred foam boosters/stabilisers include surfactants. Any suitable surfactant may be employed. A preferred surfactant is polyoxyethylene cetyl-stearyl ether, such as that commercially available as Procol LA-23. The foam stabiliser/foam booster is suitably present in an amount to impart the desired properties to the mousse. For example, the amount of foam stabiliser may be selected to give a mousse which is sufficiently stable to allow application to the skin, yet sufficiently unstable to allow the mousse to collapse quickly to form the base lubricant, once the user starts to massage the mousse into the skin. Suitable amounts of foam stabiliser/booster include, but are not limited to, amounts of about 1% w/w and below.

A pH adjuster may be included in the composition. The pH adjuster is suitably included in an amount sufficient to adjust the pH to a pH suitable for application to the skin, including the skin of the genital area. Preferably, the pH adjuster is present in an amount suitable to adjust the pH of the composition to from about pH 3.8 to about pH 4.8. Any suitable pH adjuster may be used. Preferred pH adjusters include sodium hydroxide, potassium hydroxide, calcium hydroxide, triethanolamine, ethanolamine, amnino methyl propanol, and citric acid. Other organic acids may also be suitably used. It is highly preferred that the pH adjuster is selected for compatability with the thickener.

Optionally, the composition further comprises one or more flavourings. Any suitable flavouring can be used. Preferably, the composition further comprises a preservative. Any suitable antimicrobial and/or antifungal preservative may be used. Suitable preservatives include, but are not limited to, benzoic acid, parabens such as methyl paraben and propylparaben, phenxyethanol, potassium sorbate, imidazolidinyl urea, sodium benzoate, isothiazolines and the like. Flavourings and preservatives may be present in any suitable amounts. Typically, preservatives are present in an amount less than about 0.5% w/w, for example from about 0.1% w/w to about 0.4% w/w.

A preferred composition comprises the following ingredients (amounts are by percentage weight of the total composition):

| Ingredient Function | % w/w |
|---|---|
| Humectant/Slip Agent | 10.00 - 30.00 |
| Thickener | 0.05 - 5.00 |
| Emollient/Moisturiser | 0.00 - 0.50 |
| Anti-tack Agent | 0.00 - 1.00 |
| Foam Booster/Stabiliser | 0.00 - 1.00 |
| pH Adjuster | To give desired pH |
| Flavours | 0.00 - 0.50 |
| Preservatives | 0.10 - 0.40 |
| Propellant | 0.00 - 10.00 |
| Carrier Phase | To 100 |

We have found that the 2-in-1 lubricant mousse compositions of the invention have particularly good properties. In particular, the 2-in-1 lubricant mousse compositions have particularly good properties in terms of viscosity and lubricity.

The 2-in-1 lubricant mousse compositions are of a suitable viscosity for use both as a personal lubricant and a massage lubricant. The viscosity of the lubricant base formulation is high enough that in use, once the mousse has collapsed and the composition is converted into the lubricant base formulation, it does not run off the skin, but remains in the area of application. The lubricant can therefore be massaged in where it has been applied. In comparative tests, the 2-in-1 lubricant mousse compositions were found to have significantly higher viscosities in use than the known 2-in-1 lubricant mousse products (Replens®).

Viscosities were determined using a Brookfield Viscometer, spindle SSA88, speed 100rpm. Mousses were fully degassed prior to viscosity measurement.

| Lubricant | Viscosity (cP) |
|---|---|
| 2-in-1 lubricant mousse (Example 1) | 1044 |
| Replens® Intimate Options Massage and Personal Lubricant Mousse | 13 |
| Replens® Intimate Options Personal Lubricant Mousse | 14 |

It can be seen that the degassed viscosity of the massage and personal lubricant mousse composition of the invention is higher than that of the known products (Replens®). We have found that the viscosity of the Replens® products is too low to be suitable for use as both a personal lubricant and massage lubricant because, in use, the product tends to run off the skin once the mousse breaks down. In contrast, the composition of the invention has a high enough viscosity that it does not run off the skin once the mousse has broken down, so it can be massaged in where it is applied.

The compositions also have superior lubricity properties to the known 2-in-1 lubricant mousses, both in mousse form and in the form of the lubricant base (i.e. after the mousse has collapsed, or converted to the lubricant base).

Lubricity was measured using a Texture Analyser instrument (Stable Micro Systems Ltd), by the following method. 4.5g of degassed lubricant was placed on the stationary surface of the Texture Analyser, approximating the area that the mobile sledge of the instrument would cover. The degassed lubricant was smoothed, and the sledge was fixed in position and the vertical load (300g or 500g vertical load cell) was placed on the sledge. The instrument was then set to pull the sledge back and forth across the lubricant at a sweeping speed of4.00mm/s over a distance of 7.5cm. The results for the degassed lubricant are summarised below:

| Lubricant | Initial force (N) | Time to failure (s) |
|---|---|---|
| 2-in-1 lubricant mousse (Example 1) | 0.26 | 827 |
| Replens® Intimate Options Personal Lubricant Mousse | 0.37 | 28 |
| Replens® Intimate Options Massage and Personal Lubricant Mousse | 0.30 | 150 |

The initial force measurement is a measure of static friction. This figure therefore indicates the force required to start the sledge moving. A lower value indicates a lower force required to start the sledge moving, that is, lower static friction. A lower initial force value therefore indicates higher lubricity. Lower initial force values are therefore desirable.

The time to failure is the time taken for the friction force to show a dramatic increase during the measurement cycle, that is, the time taken for the lubricant to stop providing lubrication. Time to failure therefore indicates the longevity of the lubrication provided by the lubricant. Higher times to failure are advantageous.

The above results illustrate that compositions of the invention have superior lubricity properties compared to the known 2-in-1 lubricant mousse products (Replens®). The compositions provide higher lubricity, as indicated by the lower initial force value compared to the Replens ® products. They also have a significantly longer time to failure, which indicates that they provide much longer lived lubrication.

In addition to the degassed lubricity measurements discussed above, we have measured the lubricity (in terms of initial force and time to failure) of the composition of the invention in mousse form. The following measurements were made using the method described above, employing a Texture Analyser instrument. However, measurements were made on the compositions in mousse form, rather than in degassed form.

| Lubricant Mousse | Initial Force (N) | Time to Failure (s) |
|---|---|---|
| 2-in-1 lubricant mousse (Example 1) | 0.56 | 495 |
| Replens® Intimate Options Massage and Personal Lubricant Mousse | 0.84 | 168 |

It is apparent that the composition of the invention provides superior lubricity, as indicated by the lower initial force measurement. The longevity (or time to failure) of the lubricity provided by the composition of the invention is also superior to that provided by the known product (Replens®).

We have found that the method of formulating the compositions is important. The order in which the ingredients of the composition are mixed can, to some extent, determine the properties of the composition. Accordingly, the ingredients are suitably mixed in a manner which results in a composition having optimum properties.

Preferably, the manufacturing method comprises addition of humectant to the carrier phase; followed by addition of a solution (premix 1) comprising preservative, thickener and further humectant; followed by addition of pH adjuster; followed by addition of a solution (premix 2) comprising further humectants and optionally, one or more ingredients selected from emollient, foam stabiliser and antitack agent; followed by pH adjustment. It is preferred that the solvent for the premix 1 and premix 2 solutions comprises the carrier phase.

Where the thickener comprises an alginate, such as for example propylene glycol alginate, the alginate is preferably added after any other high molecular weight ingredients (for example after any other high molecular weight thickeners and/or humectants and slip agents). Preferably, the alginate, such as propylene glycol alginate, is added after any other high molecular weight ingredients have been dissolved, mixed together and neutralised.

The following Examples illustrate the invention:

### Examples 1 and 2

| Ingredient function | Ingredient | %w/w | |
|---|---|---|---|
| | | Example 1 | Example 2 |
| Humectants and slip agents | Propylene glycol | 20.000 | 20.000 |
| | Polyglycol 35000 S¹ | 0.500 | 0.500 |
| | Kelcoloid™ S² | 1.000 | 1.000 |
| Thickeners | Natrosol® 250 M³ | 0.500 | 0.500 |
| | Natrosol® 250 L³ | 0.500 | 0.500 |
| Emollient/moisturiser | Aloe Vera 200x SD | 0.100 | 0.100 |
| Antitack agent | Microslip 519⁴ | 0.100 | - |
| | Dow Corning 193 Fluid⁵ | - | 0.500 |
| Foam booster/stabliser | Procol LA-23⁶ | 0.100 | 0.100 |
| pH adjuster | Sodium hydroxide | 0.029 | 0.038 |
| Flavour | Kangaroo | 0.100 | 0.100 |
| Preservative | Benzoic Acid | 0.150 | 0.150 |
| Propellant | Butane | 5.000 | 5.000 |
| Carrier Phase | Water | 71.921 | 71.512 |

| | | | |
|---|---|---|---|
| 1= PEG 35,000 2 = Propylene glycol alginate 3 = hydroxyethylcellulose 4 = micronised PTFE 5 = PEG-12 dimethicone 6 = PEG-23 lauryl ether | | | |

Polyglycol 35000S is available from Clariant GmbH; Kelcoloid® S is available from ISP Alginates Inc; Natrosol® 250M and 250L are available from Hercules Inc; Microslip 519 is available from Micro Powders Inc; Dow Corning 193 Fluid is available from Cow Corning Corp; Kangaroo flavour is available from Bell Flavors and Fragrances GmbH.

### Example 3

The compositions of Examples 1 and 2 are manufactured as follows.

Water is added to the main vessel. Polyglycol 3500S is slowly stirred into the water until fully dissolved. Benzoic acid, Natrosol 250L and Natrosol 250M are premixed in propylene glycol. When fully dissolved, the premix is added to the main vessel. Sodium hydroxide is premixed in water and added to the main vessel with stirring until the mix is clear and uniform. The aloe vera, Kelcoloid S, Procol LA-23 and Microslip 519 are premixed in propylene glycol and stirred until dissolved. The premix is added to the main vessel and stirred until clear and uniform. The pH is adjusted to 3.80 to 4.0.

## Claims

1. A mousse comprising a propellant and a lubricant composition capable of being formed into a mousse, which composition is suitable for use as both a massage lubricant and a personal lubricant, wherein the lubricant composition comprises a lubricant base which is a gel, from 0.5% w/w to 5% w/w thickener comprising propylene glycol alginate, from 10% w/w to 30% w/w of one or more humectants / slip agents, and a carrier.

2. A mousse according to claim 1, wherein the lubricant composition comprises a lubricant base having a degassed viscosity of from 0.05 Pa.s to 2 Pa.s (50 cP to 2000 cP), or from 0.5 Pa.s to 2 Pa.s (500 cP to 2000 cP), or from 0.5 Pa.s to 1.5 Pa.s (500 cP to 1500 cP), or/and the degassed lubricant has an initial force value as defined herein of less than 0.30 N.

3. A mousse according to any preceding claim wherein the degassed lubricant has a time to failure value, as defined herein, of 500 s or greater.

4. A mousse according to any preceding claim wherein the humectant/slip agent functions as both a slip agent and a humectant.

5. A mousse according to any preceding claim wherein the carrier comprises an aqueous carrier.

6. A mousse according to any preceding claim wherein the humectant/slip agent comprises a polyhydric alcohol, preferably a glycol, more preferably a glycol comprising a polyethylene glycol with molecular weight of 20 kDa or above or polypropylene glycol with molecular weight of 20 kDa or above.

7. A mousse according to any preceding claim further comprising one or more preservatives, moisturisers, anti-tack agents, pH adjusters, flavourings, foam boosters and/or foam stabilisers.

8. A mousse according to any preceding claim which comprises the following ingredients:
| Ingredient Function | % w/w |
|---|---|
| Humectant/Slip Agent | 10.00 - 30.00 |
| Thickener | 0.05 - 5.00 |
| Emollient/Moisturiser | 0.00 - 0.50 |
| Anti-tack Agent | 0.00 - 1.00 |
| Foam Booster/Stabiliser | 0.00 - 1.00 |
| pH Adjuster | To give desired pH |
| Flavours | 0.00 - 0.50 |
| Preservatives | 0.10 - 0.40 |
| Propellant | 0.00 - 10.00 |
| Carrier Phase | To 100 |

9. A method of making a massage and personal lubricant composition, comprising providing a lubricant composition as defined in any one of claims 1 to 8; filling the composition into a container; and pressurising the container with propellant.

10. A method as defined in claim 9 wherein the alginate is added to the lubricant composition after any other components with molecular weight of 20kDa or above have been added.

11. A method as defined in claim 10 wherein the alginate is added to the lubricant composition after any other ingredients with molecular weight of 20kDa or above have been dissolved in carrier, mixed together and neutralised.

12. A container comprising a propellant and a lubricant composition capable of being formed into a mousse, which composition is suitable for use as both a massage lubricant and a personal lubricant, wherein the lubricant composition comprises a lubricant base which is a gel, from 0.5% w/w to 5% w/w thickener comprising propylene glycol alginate, form 10% w/w to 30% w/w of one or more humectants/slip agents, and a carrier.

13. A container according to claim 12, wherein the lubricant composition is the lubricant composition as defined in any of claims 2 to 8.

## Patentansprüche

1. Schaum, umfassend ein Treibmittel und eine Gleitmittelzusammensetzung, die fähig ist, zu einem Schaum geformt zu werden, wobei die Zusammensetzung für die Verwendung sowohl als Massagegleitmittel als auch als Körpergleitmittel geeignet ist, wobei die Gleitmittelzusammensetzung eine Gleitmittelgrundlage, die ein Gel ist, von 0,5 % w/w bis 5 % w/w Verdickungsmittel, umfassend Propylenglycolalginat, von 10 % w/w bis 30 % w/w von einem oder mehreren Feuchthaltemitteln/Schlüpfrigkeitsmitteln und einen Träger umfasst.

2. Schaum gemäß Anspruch 1, wobei die Gleitmittelzusammensetzung eine Gleitmittelgrundlage umfasst, die eine entgaste Viskosität von 0,05 Pa.s bis 2 Pa.s (50 cP bis 2000 cP) oder von 0,5 Pa.s bis 2 Pa.s (500 cP bis 2000 cP) oder von 0,5 Pa.s bis 1,5 Pa.s (500 cP bis 1500 cP) aufweist und/oder das entgaste Gleitmittel einen wie hierin definierten Anfangs-Kraftwert von weniger als 0,30 N aufweist.

3. Schaum gemäß einem der vorstehenden Ansprüche, wobei das entgaste Gleitmittel einen wie hierin definierten Wert der Zeit bis zum Versagen von 500 s oder mehr aufweist.

4. Schaum gemäß einem der vorstehenden Ansprüche, wobei das Feuchthaltemittel/Schlüpfrigkeitsmittel sowohl als Schlüpfrigkeitsmittel als auch als Feuchthaltemittel wirkt.

5. Schaum gemäß einem der vorstehenden Ansprüche, wobei der Träger einen wässrigen Träger umfasst.

6. Schaum gemäß einem der vorstehenden Ansprüche, wobei das Feuchthaltemittel/Schlüpfrigkeitsmittel einen polyhydrischen Alkohol umfasst, vorzugsweise ein Glycol, bevorzugter ein Glycol, das ein Polyethylenglycol mit einem Molekulargewicht von 20 kDa oder mehr oder Polypropylenglycol mit einem Molekulargewicht von 20 kDa oder mehr umfasst.

7. Schaum gemäß einem der vorstehenden Ansprüche, ferner umfassend ein oder mehrere Konservierungsmittel, Befeuchtungsmittel, Antihaftmittel, pH-Wert-Regulatoren, Aromastoffe, Schaumverstärker und/oder Schaumstabilisatoren.

8. Schaum gemäß einem der vorstehenden Ansprüche, umfassend folgende Inhaltsstoffe:
| Inhaltsstoff-Funktion | % w/w |
|---|---|
| Feuchthaltemittel/Schlüpfrigkeitsmittel | 10,00-30,00 |
| Verdickungsmittel | 0,05-5,00 |
| Weichmacher/Befeuchtungsmittel | 0,00-0,50 |
| Antihaftmittel | 0,00-1,00 |
| Schaumverstärker/-stabilisator | 0,00-1,00 |
| pH-Wert-Regulator | zum Ergeben des gewünschten pH-Werts |
| Aromastoffe | 0,00-0,50 |
| Konservierungsmittel | 0,10-0,40 |
| Treibmittel | 0,00-10,00 |
| Trägerphase | auf 100 |

9. Verfahren zum Herstellen einer Massage- und Körpergleitmittelzusammensetzung, umfassend Bereitstellen einer Gleitmittelzusammensetzung gemäß einem der Ansprüche 1 bis 8; Abfüllen der Zusammensetzung in einen Behälter; und Unterdrucksetzen des Behälters mit Treibmittel.

10. Verfahren gemäß Anspruch 9, wobei das Alginat zu der Gleitmittelzusammensetzung zugegeben wird, nachdem alle anderen Komponenten mit einem Molekulargewicht von 20 kDa oder mehr zugegeben worden sind.

11. Verfahren gemäß Anspruch 10, wobei das Alginat zu der Gleitmittelzusammensetzung zugegeben wird, nachdem alle anderen Inhaltsstoffe mit einem Molekulargewicht von 20 kDa oder mehr in Träger gelöst, zusammengemischt und neutralisiert worden sind.

12. Behälter, umfassend ein Treibmittel und eine Gleitmittelzusammensetzung, die fähig ist, zu einem Schaum geformt zu werden, welche Zusammensetzung für die Verwendung sowohl als Massagegleitmittel als auch als Körpergleitmittel geeignet ist, wobei die Gleitmittelzusammensetzung eine Gleitmittelgrundlage, die ein Gel ist, von 0,5 % w/w bis 5 % w/w Verdickungsmittel, umfassend Propylenglycolalginat, von 10 % w/w bis 30 % w/w von einem oder mehreren Feuchthaltemitteln/Schlüpfrigkeitsmitteln und einen Träger umfasst.

13. Behälter gemäß Anspruch 12, wobei die Gleitmittelzusammensetzung die Gleitmittelzusammensetzung gemäß einem der Ansprüche 2 bis 8 ist.

## Revendications

1. Mousse comprenant un propulseur et une composition de lubrifiant capable d'être mise sous forme de mousse, laquelle composition convient pour une utilisation à la fois en tant que un lubrifiant pour massage et en tant que lubrifiant intime, dans laquelle la composition de lubrifiant comprend une base de lubrifiant qui est un gel, de 0,5% en poids à 5% en poids d'un épaississant comprenant de l'alginate de propylèneglycol, de 10% en poids à 30% en poids d'un ou plusieurs humectants / agents de glissement, et un véhicule.

2. Mousse selon la revendication 1, dans laquelle la composition de lubrifiant comprend une base de lubrifiant ayant une viscosité dégazée allant de 0,05 Pa.s à 2 Pa.s (50 cP à 2000 cP), ou de 0,5 Pa.s à 2 Pa.s (500 cP à 2000 cP), ou de 0,5 Pa.s à 1,5 Pa.s (500 cP à 1500 cP), ou/et le lubrifiant dégazé possède une force initiale, selon la définition donnée ici, qui est inférieure à 0,30 N.

3. Mousse selon l'une quelconque des revendications précédentes dans laquelle le lubrifiant dégazé possède une durée de fonctionnement avant défaillance, selon la définition donnée ici, qui est de 500 s ou plus.

4. Mousse selon l'une quelconque des revendications précédentes dans laquelle l'humectant/agent de glissement fait office à la fois d'agent de glissement et d'humectant.

5. Mousse selon l'une quelconque des revendications précédentes dans laquelle le véhicule comprend un véhicule aqueux.

6. Mousse selon l'une quelconque des revendications précédentes dans laquelle l'humectant/agent de glissement comprend un alcool polyhydroxylé, de préférence un glycol, de façon plus préférée un glycol comprenant un polyéthylèneglycol ayant une masse moléculaire de 20 kDa ou plus ou un polypropylèneglycol ayant une masse moléculaire de 20 kDa ou plus.

7. Mousse selon l'une quelconque des revendications précédentes comprenant en outre un ou plusieurs conservateurs, hydratants, agents anti-poisseux, agents d'ajustement du pH, arômes, renforçateurs de mousse et/ou stabilisants de mousse.

8. Mousse selon l'une quelconque des revendications précédentes qui comprend les ingrédients suivants :
| Fonction de l'ingrédient | % en poids |
|---|---|
| Humectant/Agent de glissement | 10,00 - 30,00 |
| Epaississant | 0,05 - 5,00 |
| Emollient/Hydratant | 0,00 - 0,50 |
| Agent anti-poisseux | 0,00 - 1,00 |
| Renforçateur de mousse/Stabilisant | 0,00 - 1,00 |
| Agent d'ajustement du pH | jusqu'au pH souhaité |
| Arômes | 0,00 - 0,50 |
| Conservateurs | 0,10 - 0,40 |
| Propulseur | 0,00 - 10,00 |
| Phase de véhicule | à 100 |

9. Procédé de fabrication d'une composition de lubrifiant de massage et intime, comprenant le fait de se procurer une composition de lubrifiant telle que définie dans l'une quelconque des revendications 1 à 8, de remplir un récipient avec la composition et de pressuriser le récipient avec le propulseur.

10. Procédé selon la revendication 9 dans lequel l'alginate est ajouté à la composition de lubrifiant après que tous les autres constituants ayant une masse moléculaire de 20 kDa ou plus ont été ajoutés.

11. Procédé selon la revendication 10 dans lequel l'alginate est ajouté à la composition de lubrifiant après que tous les autres ingrédients ayant une masse moléculaire de 20 kDa ou plus ont été dissous dans le véhicule, mélangés les uns aux autres et neutralisés.

12. Récipient comprenant un propulseur et une composition de lubrifiant capable d'être mise sous forme de mousse, laquelle composition convient pour une utilisation à la fois en tant que lubrifiant pour massage et en tant que lubrifiant intime, dans lequel la composition de lubrifiant comprend une base de lubrifiant qui est un gel, de 0,5% en poids à 5% en poids d'un épaississant comprenant de l'alginate de propylèneglycol, de 10% en poids à 30% en poids d'un ou plusieurs humectants/agents de glissement, et un véhicule.

13. Récipient selon la revendication 12, dans lequel la composition de lubrifiant est la composition de lubrifiant telle que définie dans l'une quelconque des revendications 2 à 8.
